# EUROPEAN PATENT SPECIFICATION

(11) **EP 1 370 680 B1**
(45) Date of publication and mention of the grant of the patent: **12.04.2006**
(21) Application number: 01984657.5
(22) Date of filing: 11.09.2001
(51) Int. Cl.: C12Q 1/32, C12Q 1/04, G01N 33/52

(54) **METHOD FOR THE DETECTION AND MEASUREMENT OF BIOMASS**
VERFAHREN ZUR DETEKTION UND MESSUNG DER BIOMASSE
METHODE DE DETECTION ET DE MESURE DE BIOMASSE

(30) Priority: 13.09.2000 EP 00119418
(43) Date of publication of application: 17.12.2003
(73) Proprietor: Merck Patent GmbH, 64293 Darmstadt (DE)
(72) Inventor: CHITTOCK, Roger Steward, Caerphilly CF83 8SX (GB); KAU, Paul, Pontypridd CF38 2JP (GB); WATKINS, Ian David, Cardiff CF15 8LT (GB); DAVIES, Alan Michael, Pontypridd CF37 2HB (GB)
(86) International application number: PCT/EP2001/010486
(87) International publication number: WO 2002/022854

(56) References cited:
- EP-A- 0 315 058
- US-A- 3 415 718
- US-A- 4 435 504
- BERGEL A ET AL: "ENZYMATIC AMPLIFICATION FOR SPECTROPHOTOMETRIC AND ELECTROCHEMICAL ASSAYS OF NAD AND NADH" ANALYTICAL BIOCHEMISTRY, vol. 179, no. 2, 1989, pages 382-388, XP008011963 ISSN: 0003-2697

## Description

This invention relates to the detection and measurement of biomass based upon methods for the enzymatic assay of nicotinamide adenine dinucleotide compounds.

### Background of the invention

The assessment of levels of biological material ("biomass") present in samples is of value in a wide range of applications. In some applications, the assessment is made in order to monitor processes, for example in order to determine levels of micro-organisms in fermentation processes and other microbiological cultures. In other applications, measurement of biomass gives an indirect indication of environmental hygiene status and cleanliness. This is particularly relevant in checking, for example, cleanliness of areas used for food preparation and storage and also cleanliness of areas within clinical environments. In other applications, measurement of biomass can be used to directly detect and measure the presence of unwanted micro-organisms or other biological materials in samples which would be expected to be free of such contaminants, for example, petrochemicals, lubricating oils, foodstuffs, industrial process waters and other water supplies and sources.

There are a variety of techniques available for the detection and measurement of biomass. Biomass has been estimated from the volume fraction of a solution that is enclosed within cytoplasmic membranes calculated from dielectric or capacitative properties using for example a potentiostat (GB 2 298 046). A second method measures the oxygen consumption of a solution using a suitable electrode (GB 2 280 431). Biomass can also be determined directly from the optical (US 4 893 935) or sedimentation (DE 38 19 540) properties of a suspension of micro-organisms. All these methods can only be used in applications in which the level of biomass is high, for example analysis of industrial and urban effluent, because they are relatively insensitive. These methods also depend upon expensive instrumentation.

The growth of bacterial, yeast and mammalian cells growing in fermenters and bioreactors has been estimated from the direct fluorescence of cultures, more than half of which arises from intracellular NADH and NADPH (Zabriskie, D.W. and Humphrey, A.E. "Estimation of Fermentation Biomass Concentration by Measuring Culture Fluorescence" (1978) Applied and Environmental Microbiology 35, 337-343). The method is relatively insensitive and only suitable for applications where viable cell numbers are extremely high. Since NAD(P)H, but not NAD(P), will produce a fluorescent signal the method is prone to artefacts caused by fluctuations in the intracellular NAD(P):NAD(P)H ratio as the metabolic state of the cells change. Quantification of cell numbers may also be compromised by internal screening effects, changes in medium composition (especially pH) and fluorescent compounds in the extracellular medium.

A further technique available for the detection and measurement of biomass is measurement of production of light by an enzymatic reaction utilising ATP as one substrate, as this molecule is a ubiquitous component present in biological materials of cellular origin (Hawronskyj, J.M. and Holah, J. "ATP: A universal hygiene monitor" (1997) Trends in Food Science and Technology 8, 79-84). ATP will produce light measurable by a suitable photodetector in the presence of firefly luciferase (EC 1.13.12.7) and appropriate substrates. Alternatively, the cellular enzyme adenylate kinase may be used for the detection and measurement of biomass by using firefly luciferase to measure its production of ATP when ADP is added to the sample (Squirrell, D.J. and Murphy, M.J. "Adenylate kinase as a cell marker in bioluminescent assays" (1994) in "Bioluminescence and Chemiluminescence: Fundamentals and Applied Aspects" ed. Campbell, A.K., Kricka, L.J. and Stanley, P.E. pub. Wiley and sons, Chichester, UK.).

Methods involving luminescence measurement of ATP can produce quantitative results within minutes. This represents an advantage over traditional methods involving micro-biological enumeration, such as micro-biological plate counting, which count viable micro-organisms only and requires days to produce a result, as this is too slow for many applications. Plate counting methods also measure populations of viable micro-organisms selectively, according to the culture conditions and procedures chosen. However, methods involving luminescence measurement of ATP, although relatively sensitive, require expensive instrumentation and reagents. In particular, the luminescence output from these methods cannot be assessed visually.

Alternatively, test procedures for measurement of biomass are conceivable in which the results can be assessed visually and which therefore do not necessitate the use of an instrument. A test procedure not requiring an instrument represents a potential improvement in cost-effectiveness over luminescence methods, especially for the low volume user. Additionally, such a procedure would have advantages of ease of interpretation and lower training and maintenance requirements.

Reagents that give a chemical reaction resulting in a colour change in the presence of such analytes as protein, starch or lipid have been described. The chemistry underlying these reactions is relatively simple and convenient to use. Although these colour stain reactions may have some application in the assessment of biological materials derived, for example, from foodstuffs, they are especially insensitive and are particularly unsuitable for biomass determinations in circumstances in which micro-organisms are present in significant levels within the samples, as the stains do not readily react with micro-organisms.

A further technique uses the dehydrogenase activity of micro-organisms to detect the biomass present in a sample (GB 1 603 183). Samples from a bio-reactor or effluent samples are pre-incubated under anaerobic conditions for 15 to 20 minutes with a redox indicator such as a colourless tetrazolium salt. This indicator is converted to an insoluble coloured formazan product by the various dehydrogenase enzymes present in the micro-organisms. At the end of the incubation all enzymatic activity is stopped, the formazan product extracted and measured colorimetrically to give an estimate of the biomass in the sample. This method does not incorporate amplification or recycling of the relevant substrates and is therefore again relatively insensitive, lengthy and inconvenient and is designed specifically for monitoring fermentation or the content of industrial effluent where biomass is relatively high.

Multi-enzyme linked assays have been described which produce a visible colour change (WO 94/25619) or a fluorometric signal (Hansen, E.H., Gundstrup, M. and Mikkelsen, H.S. "Determination of minute amounts of ATP by flow injection analysis using enzyme amplification reactions and fluorescence detection." (1993) Journal of Biotechnology 31, 369-380) from sub-nanomolar concentrations of ATP. Difficulties arise because these assays incorporate pathways that require two steps, both of which incorporate at least two enzyme components.

An improvement over existing colorimetric determinations of biomass would give significant benefits if it were to give a reliable determination of a compound ubiquitous in biological material combining good wide-spectrum sensitivity performance with the use of a wide range of simple and robust formulations, ease of manufacture and adaptable to a wide range of formats.

### Brief description of the Invention

The present invention relates to a method for detecting the presence of biomass in a sample based on the detection of the total pool of NAD(P) and NAD(P)H, characterised in that the total pool of NAD(P) and NAD(P)H is measured as a marker for the presence of biomass by conversion of NAD(P) in the sample to NAD(P)H enzymatically by adding an enzyme E1 and a substrate S1 for that enzyme.

In one preferred embodiment of the present invention, the presence of biomass is not only detected but the level of biomass is also measured.

Preferably, the enzyme E1 is a dehydrogenase.

In one embodiment the detection of the reduced form of the nicotinamide adenine dinucleotides is done fluorimetrically.

In another preferred embodiment of the present invention the detection or measurement is done by adding a second enzyme E2 and a second substrate S2, whereby the following reaction takes place 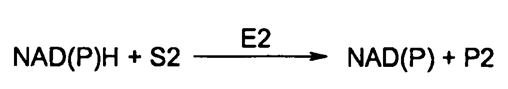 and the conversion of S2 into P2 generates a signal which is a change in the absorbance spectrum including a change in colour, a change in fluorescence, a luminescent reaction or an electrochemical reaction leading to a change in potential or current at an electrode surface. Preferably the signal is a change in colour which can be detected visually.

In another preferred embodiment, alternatively, conversion of NAD(P)H to NAD(P) may occur directly at the surface of a suitable electrode without the need for a second enzyme E2 or second substrate S2, to produce a change in potential or current at the electrode surface.

In another preferred embodiment of the present invention the substrates S1 and S2 are added to the sample in excess. Thus a cycling detection system is generated which enhances the sensitivity of the measurement. This is especially suitable for the assessment of the hygiene status of a sample.

In another preferred embodiment E1 is lactate dehydrogenase, E2 is diaphorase, S1 is lithium lactate and S2 is nitro blue tetrazolium.

In a very preferred embodiment E1 is glucose dehydrogenase, E2 is diaphorase, S1 is glucose and S2 is nitro blue tetrazolium.

In one preferred embodiment the sample is heated at a temperature between 25 and 45 °C, preferably at 35 to 45 °C so that the rate of the reaction is enhanced and detection time is minimised.

In another preferred embodiment prior to detecting or measuring the amount of biomass a detergent and optionally afterwards a neutraliser are applied to the sample. It is even possible to do one determination without the application of a detergent and one with the application of a detergent. This gives the possibility to distinguish between extra- and intracellular nicotinamide adenine dinucleotide pools.

The present invention also relates to the use of the above mentioned method for the assessment of microbial content or the hygiene status of a sample.

### Description of the drawings

Figures 1 and 2 are schemes of enzymatic reactions, more or less general, that may be utilised in the present invention.
Figure 3 is a comparison of nicotinamide adenine dinucleotide compounds present in food residues measured by the current invention and ATP measured by the HY-LiTE® hygiene monitoring system present in the same food residues.
Figure 4 is an example of the current invention used to monitor the growth of a bacterial culture.
Figure 5 is a schematic, illustrative view of a lateral flow test strip for use according to the present invention.

Abbreviations used in figures, tables and elsewhere have the following meanings:
- ADP: adenosine-5'-diphosphate
- ATP: adenosine-5'-triphosphate
- BSA: bovine serum albumin
- DTAB: dodecyl trimethyl ammonium bromide
- EDTA: ethylenediaminetetraacetate
- G6P: glucose-6-phosphate
- G6PDH: glucose-6-phosphate dehydrogenase
- INT: iodonitrotetrazolium violet
- NAD: nicotinamide adenine dinucleotide (oxidised form)
- NAD(P): nicotinamide adenine dinucleotide and nicotinamide adenine phosphate (oxidised form)
- NAD(P)H: nicotinamide adenine dinucleotide and nicotinamide adenine phosphate (reduced form)
- NADH: nicotinamide adenine dinucleotide (reduced form)
- NADP: nicotinamide adenine dinucleotide phosphate (oxidised form)
- NADPH: nicotinamide adenine dinucleotide phosphate (reduced form)
- NBT: nitro blue tetrazolium
- TVC: total viable colonies

### Description of the invention

The current invention relates to the assay of nicotinamide adenine dinucleotides such as NAD, NADH, NADP and NADPH for the detection and measurement of biomass. These compounds are found as components of cells and free in the environment.

The measurement of NAD(P)H alone for the detection of biomass is prone to artefacts due to the changing NAD(P) / NAD(P)H ratio. It has been found that these problems are overcome by converting all NAD(P) in the sample to NAD(P)H and afterwards detecting NAD(P)H as a marker for the total pool of NAD(P) and NAD(P)H in the sample to offer a simple and sensitive method for the detection of biomass.

An advantage associated with the invention is that nicotinamide adenine dinucleotides can readily be detected and measured by colorimetric enzymatic assays using formulations that are much simpler than those required for colorimetric enzymatic assays of markers such as ATP and ADP. A very wide range of enzymes and substrates can be employed. As an additional advantage, measurement of these compounds can be applied successfully to the determination of biomass in samples containing significant levels of micro-organisms and so these measurements are more suited to such applications than chemical stains for determination of protein, lipid or starch.

As a further advantage, nicotinamide adenine dinucleotides can also readily be measured by electrochemical and direct spectroscopic assays, unlike current markers of biomass such as ATP.

According to the method of the current invention as shown in reaction scheme I, nicotinamide adenine dinucleotides in the oxidised form, for example NAD and NADP, present in a sample are converted to nicotinamide adenine dinucleotides in the reduced form, for example NADH and NADPH, by a suitable enzyme (E1) with its second substrate (S1).

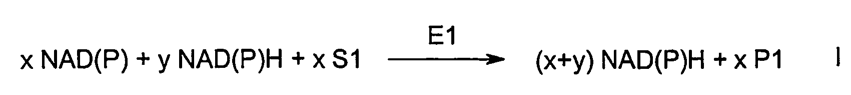

A suitable enzyme E1 is any enzyme that converts NAD(P) to NAD(P)H. In a preferred embodiment this enzyme is a dehydrogenase, for example glucose-6-phosphate dehydrogenase.
NADH and NADPH present in the sample will also be detected by this assay. This gives a significant benefit compared to assays such as the UV fluorescence of bacterial and cell cultures in which only the reduced form is measured because the ratio of oxidised : reduced nicotinamide compounds changes depending on the metabolic state of the cell. The current invention is not subject to this potential problem because both oxidised and reduced forms ((x+y) NAD(P)H according to scheme I) can be detected.

The detection can be done directly by measuring the fluorescence of the NAD(P)H in the sample or indirectly by generating a detectable signal P2 using an additional enzymatic or chemical reaction, e.g. as depicted in reaction scheme II:

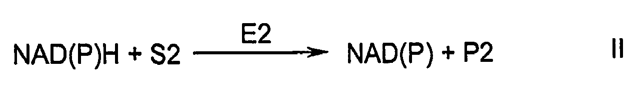

The signal from these reactions may be a change in colour, other spectral properties, fluorescence properties, luminescence or electrochemical potential. Measurements of signals generated by the determination of nicotinamide adenine dinucleotides may be made respectively by the use of reflectometer or spectrophotometer with a colour signal, according to whether the coloured product is in soluble or insoluble form, a fluorimeter with a fluorescent signal, a luminometer with a luminescent signal or a potentiometer with an electrochemical potential or amperometric signal.

In order to improve the sensitivity, in another preferred embodiment of the present invention, NAD(P) and NAD(P)H present in the sample are both recycled.

As could be seen from reaction scheme II, in terms of a chemical or enzymatic means of detection, NADH and NADPH are re-converted to NAD or NADP by reaction with a suitable donor molecule S2, either directly, via a suitable chemical mediator or via an enzyme catalysed reaction. Reduction of this donor, the second product of the dehydrogenase or the reduced form of the nicotinamide adenine dinucleotide produced leads to a signal P2 used as an indicator of the presence of biomass.

The NAD(P) generated in this reaction is then recycled into NAD(P)H again by using the same enzyme E1 that was used to originally convert the NAD(P) present in the sample into NAD(P)H. As a consequence a simple cycling system is generated which improves the sensitivity of the assay and is especially suitable for the detection of low amounts of biomass. To make this cycling system work properly it is necessary to provide an excess of the enzyme substrates S1 and S2.

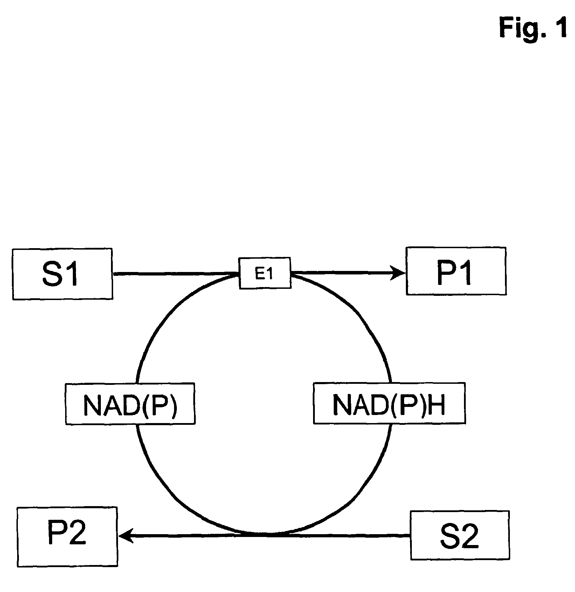

A scheme of a cycling system capable of recycling between oxidised and reduced forms of nicotinamide adenine dinucleotide is shown in figure 1. By the action of enzyme E1 NAD or NADP is converted to NADH or NADPH with corresponding oxidation of the enzyme's second substrate S1, present in excess. The NAD(P)H formed is recycled to NAD(P) with a corresponding reduction of a suitable donor molecule, S2, present in excess. Detection of NAD(P) and NAD(P)H present in the original sample may be made from changes in the properties of this donor molecule when it is reduced or from the changes in substrate S1 when it is oxidised. This may be a change in absorbance spectrum including a change in colour, a change in fluorescence, a luminescent reaction or an electrochemical reaction leading to a change in potential at an electrode surface. The reaction between NADH and S2 may occur directly or may be mediated by a chemical intermediate or may be catalysed by an enzyme E2.

Alternatively the NADH in a sample may be detected directly by an electrochemical reaction catalysed by an appropriate enzyme, either with a dehydrogenase or other enzyme to recycle NAD or alone, using an appropriate detector to achieve the necessary sensitivity.

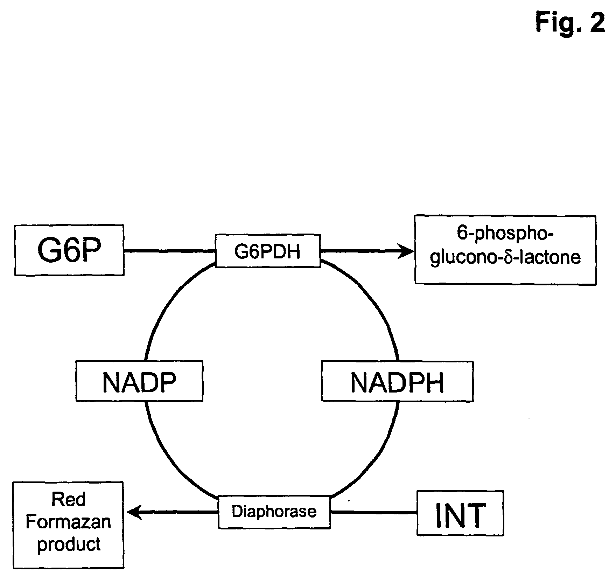

A more specific example of the invention is illustrated in figure 2. Recycling is achieved by two enzymes linked to a colour change on reduction of the donor molecule suitable for detection by eye. Enzyme E1 is yeast glucose-6-phosphate dehydrogenase (EC 1.1.1.49) which produces 6-phosphoglucono-δ-lactone and NADPH from D-glucose-6-phosphate and NADP. Enzyme E2 is diaphorase (EC 1.8.1.4) which produces a formazan product and NADP from NADPH and a tetrazolium salt, for example p-iodonitrotetrazolium violet (INT). The progress of the reaction indicating the presence of NADP and NADPH in the original sample is monitored by the change in colour on production of formazan, monitored by eye as a red colour or spectrophotometrically at 500 nm.

Many dehydrogenases and the corresponding substrates may be used for the reduction of NAD(P) in place of glucose-6-phosphate dehydrogenase and D-glucose-6-phosphate. For example glucose-6-phosphate dehydrogenase from *Leuconostoc mesenteroides* (EC 1.1.1.49, specific for NAD as well as NADP), glucose dehydrogenase (EC 1.1.1.47), lactate dehydrogenase (EC 1.1.1.27), pyruvate dehydrogenase (EC 1.2.4.1), malate dehydrogenase (EC 1.1.1.37), aldehyde dehydrogenase (EC 1.2.1.5) and alcohol dehydrogenase (EC 1.1.1.1).

In an equivalent way, many other enzymes and their corresponding suitable substrates may be used in place of diaphorase for the oxidation of NAD(P)H and the generation of a detectable signal.

It is for example possible to produce colour from NADH using liver alcohol dehydrogenase and a nitrosamine substrate but as the substrate is toxic it should not be used for the detection of biomass in e.g. food preparation or clinical environments.
In addition, diaphorase can be replaced by a chemical mediator such as Meldola's blue which is first reduced by NADH and then reoxidised by NBT with formation of the coloured formazan salt. NADH will react directly with NBT to produce a coloured formazan but this reaction is very slow (~hours). Other compounds that can react with NAD(P)H to give a colour change are DIP (dichlorophenolindophenol), methylene blue and thionine.

Luminescent detection of NADH is possible using the enzyme bacterial luciferase.
For the specific detection of NADPH, NADPH can be converted to NADP by glutathione oxidase with reduction of glutathione which can be detected by its reaction with DTNB (5,5'-dithiobis-(2-nitrobenzoic acid)).
If NADH is detected amperometrically the conversion of NAD(P)H to NAD(P) can occur directly at the electrode surface so no second enzyme is necessary.

Suitable substrates e.g. for the reaction with diaphorase are tetrazolium salts which give a coloured formazan product. Examples are NBT or MTT (3-(4,5-dimethylthiazol-2-yl)-2,5-diphenyl-2-tetrazolium bromide).

The enzyme reactions can be performed in any standard buffer that is compatible with the enzymes employed. One example of a preferred buffer system is a buffer consisting of 200 mM Tris, 50% glycerol, 0.01 % proclin, 0.3% Tween 80, pH 8 and a substrate formulation consisting of 10 mM sodium citrate buffer pH 6, 0.01 % proclin and 2% (w/v) alpha-cyclodextrin.

Premature colour formation may be prevented by preparing the formulation as two reagent pre-mixes which are mixed with sample to initiate the reaction.

The measurement of NADH and NADPH using a high concentration of a purified dehydrogenase in the assay formulation of the invention provides superior sensitivity to colorimetric procedures based on, for example, the assay of the endogenous dehydrogenase activity of micro-organisms by reduction of a redox indicator (GB 1 603 183).

Nicotinamide adenine dinucleotides can be detected and measured as a marker for biomass utilising a very wide range of test formats. Appropriate test formats include microtiter wells or test tubes as well as swabs, test strips, sensors, tubes and also filter and membrane surfaces. In particular, biomass present in fluid samples can be concentrated by filtration on to the surface of appropriate membranes before assay.

For example, surfaces may be sampled using a test strip comprising of a plastic support onto which an appropriate paper pad is attached at one end or alternatively the test strip could be replaced with a conventional swab comprising a plastic shaft onto which a cotton or synthetic material is wound tightly at one end to form a head or alternatively a similar appropriate sampling device. The sampling device may be wetted by an appropriate wetting solution before sampling if necessary.

As a further aspect within the current invention, nicotinamide adenine dinucleotides can be detected and measured as a marker for biomass using procedures within which the rate of the reaction chemistry is enhanced by the application of heat. As it is known that enzyme activity is proportional to temperature and that enzymes are destroyed by the application of heat, the preferred temperature for the method of the present invention is between 20 and 45 °C. Ideal temperatures for the enhancement of the reaction are between 25 and 45 °C, preferably between 35 and 45°C.

As a further aspect within the current invention, nicotinamide adenine dinucleotides can be detected and measured as a marker for biomass using procedures within which the biochemical reactions are stopped by addition of a suitable reagent after a defined period. The reaction might be stopped chemically or by washing the reagents off the sampling device. If e.g. a coloured formazan product is generated on a test strip and the test strip is washed with water, the insoluble formazan product sticks to the test strip while the other reagents (enzymes etc.) are washed off.

As a further aspect within the current invention, nicotinamide adenine dinucleotides can be determined after release from samples based on cellular materials for example micro-organisms using an appropriate physical or chemical treatment such as detergent extraction. Extraction reagents such as detergents in a suitable formulation may be included in mixtures with other assay components or added separately without harm to the assay biochemistry/chemistry.

In particular, in this invention it is proposed that intracellular nicotinamide adenine dinucleotides are extracted from bacterial and other microbial cells using a cationic detergent, for example a quaternary ammonium compound e.g. DTAB, followed by the use of a neutraliser like alpha or beta cyclodextrin to neutralise the detergent. The released nicotinamide adenine dinucleotides are then available for measurement without the potential interference of the cationic detergent (WO 92/12253). Non-ionic or zwitterionic detergents may also be used for lysis of mammalian or other eukaryotic cells in order to extract intracellular nicotinamide adenine dinucleotides.

As a further aspect within the current invention protocols can be considered in which background NAD(P)(H) from eukaryotic cells and free in the sample is extracted by the use of mild procedures such as non-ionic detergents and then destroyed by a suitable NADase, prior to extraction of NAD(P)(H) from microbial sources by the use of stronger extractants such as cationic detergent. In this way microbial NAD(P)(H) can be selectively measured in the presence of elevated NAD(P)(H) from background or eukaryotic cellular sources.

As a further aspect within the current invention, by the use of appropriate measurements and controls, it is possible to undertake comparative determinations of the levels of nicotinamide adenine dinucleotides. For example, comparative determination of the levels of nicotinamide adenine dinucleotides in a sample with and without extraction by detergent can be used to quantify the level of nicotinamide adenine dinucleotides present specifically within cellular materials, such as bacteria and can therefore be used for approximate quantification of cells. As another example, the determination of the changes with time in levels of nicotinamide adenine dinucleotides can be used to detect growth and to define and measure growth curves of living cells in a sample [see example 4]. This is an important aspect in which convenience of handling and also sensitivity presents an advantage over optical methods whilst time is a considerable advantage over plate count methods. It is also possible to obtain a colorimetric signal, for example, to detect growth in bacterial broth cultures before it is possible to see growth visually by eye.

As an aspect of the current invention, nicotinamide adenine dinucleotides can be detected and measured as a marker for biomass from a very wide range of samples. This includes
- Food manufacture, preparation, handling, storage surface and liquid samples and other samples for hygiene testing.
- Food stuffs and food materials (including dairy and beverages)
- Process water, cooling tower and heat exchange system, pipework and other industrial water samples
- Oil, mineral oil, lubricant, petrochemical samples
- Cleaning samples and solutions
- Shower, bath, toilet, swimming pool samples
- Hospital and clinical / clinic / dentistry samples
- Medical and veterinary samples
- Air samples in general; aerosols in general
- Samples from building and domestic environments in general
- Biofilms (films of micro-organisms present on / adhering to surfaces)
- Airborne micro-organisms [germ warfare]
- Environmental samples
- Microbiological broth cultures
- Microbiological fermentations [beer; yoghurt; etc]
- Cell tissue culture
- Fermentation starter cultures
- Samples from bioremediation and water treatment systems
- Assays using inhibition of cell growth or bacterial growth to assess pollutants or pharmacologically active or other inhibitory compounds
- Measurement of bacterial and algal growth in freshwater rivers and lakes.
- Samples tested for the efficacy of biocide treatment.

Disclosed is a test kit at least comprising the reagents necessary for performing the enzymatic conversion of NAD(P) to NAD(P)H, preferably also the reagents necessary to detect NAD(P)H by generating a signal. That means the reagents comprise the enzymes E1 and E2 and the substrates S1 and S2 in one or more containers. In one preferred embodiment the test kit comprises a wetting solution (e.g. water or a mixture of water with an alcohol like isopropanol or a mixture of water with a sterilising agent like sodium hypochlorite), a substrate solution (e.g. comprising NBT, glucose, a suitable buffer solution etc.) and an enzyme solution (e.g. diaphorase, glucose dehydrogenase, a suitable buffer solution etc). In another preferred embodiment the test comprises one container with all enzymes and substrates.

Preferably, the test kit also contains a sampling device in form of a test strip, a swab, an absorbent material on a solid support etc. In addition, further aids, like standard solutions, a written protocol of the detection method, a standard colour chart etc. might be included.

Disclosed is a lateral flow test device for detecting the presence of biomass in a sample according to the method of the present invention. The test device comprises a test strip with a dry matrix material capable of transporting a liquid therealong by capillarity and having at least a start zone for receiving said sample and a reaction zone having at least one kind of enzyme immoblilized therein.

The start zone is preferably situated at one end of the test strip. The sample to be analysed is applied to the start zone. In a preferred embodiment, the start zone is a sample pad composed of an absorbent paper. Glass fibre materials, fibrous plastic materials such as Porex® sheet materials (Porex Corporation), and non-woven fabrics comprising such materials as viscose and polyester may also be used. Preferably such materials should be in a single sheet or layer. The volume capacity of the sample pad defines the initial sample uptake of the device. The sample pad also acts as a filter to help to prevent unwanted particulate materials from reaching the reaction zone. The material of the start zone helps to control the release of any impregnated mobilisable components of the assay to the rest of the device and should therefore be compatible with this purpose. Liquid samples can be added to the start zone as single drops e.g. by using a pipette or by dipping the start zone in the liquid sample. The start zone can comprise components which are dried onto it such as components of the enzyme assay and/or chemicals to prevent ("block") non-specific binding effects at the reaction zone and/or to chemicals to enhance hydrophilic properties, rehydration of assay components and lateral flow characteristics and/or analyte release agents and/or extractants, preferably detergent extractants.

The reaction zone might be located directly adjacent to the start zone or further down the test strip. It contains one or more components of the enzyme assay in a stable state, in particular at least one kind of enzyme immobilised to the reaction zone. Additional components might be additional immobilised enzymes and/or immobilised or dried enzyme substrates, co-substrates, co-factors etc. The reaction zone may be a pad or a membrane chosen to be suitable for procedures to non-covalently or covalently immobilise protein without denaturation and also for the lateral flow characteristics of the material. The reaction zone may be composed of a nitrocellulose membrane with a high protein binding capacity. Such membrane may also incorporate cellulose acetate. The pad or membrane may be treated before and/or after enzyme immobilisation in order to minimise unwanted effects such as non-specific binding of analyte and/or assay components and/or in order to enhance hydrophilic properties and lateral flow. It is on the reaction zone that the reactions comprising the assay occur and the product indicating the presence of the analyte in the original sample is formed.

The test strip preferably further comprises a reagent zone, typically composed of materials similar to those employed for the sample pad, preferably composed of absorbent paper or of glass fibre or of polyethylene fibre or of polyester, localised between the start zone and the reaction zone or partially overlapping with one or both of these zones. The reagent zone contains one or more components of the assay dried in stable state such as enzyme substrate components and/or chemicals to prevent ("block") non-specific binding effects at the reaction zone and/or chemicals to enhance hydrophilic properties, rehydration of assay components and lateral flow characteristics and/or analyte release agents and/or extractants, preferably detergent extractants instead or in addition to the start zone.

Preferably, the test strip incorporates a wick or a comparable means which draws liquid sample from the matrix of the test strip through the device and therefore drives the capillary flow from the sample pad. Flow of solution into the wick ensures a sustained flow from the sample and reagent pads across the reaction site of the immobilised enzymes to maximise the amount of product formed. The wick is typically composed of materials similar to those employed for the sample pad. In a preferred embodiment, the wick is composed of an absorbent paper. The assay reaches endpoint only when the volume capacity of the absorbent pad is filled, assuming that sample volume is not limiting.

The test strip further comprises a plastic case or other housing to provide additional strength and rigidity to the device and to facilitate handling of the device without contaminating it. Preferably, the test strip is only partially covered by the housing. The start zone is preferably not covered by the housing to ensure easy application of the sample.

The various absorbent zones of the test strip are all in the same plane, allowing capillary flow of a liquid sample between the zones. In the process, any component(s) deployed in dried state on the test strip, such as detergent to extract the analyte from matrices within the sample, are reconstituted into solution. All zones may contain further reagents like stabilising agents or buffers which e.g. support the storage of the components of the enzyme reaction or provide reaction conditions (e.g. pH) that are suitable for the enzyme reaction. For some applications it might be favourable to combine the enzyme reaction with a chemical reaction to e.g. improve the generation of a detectable signal. In this case, the components of the chemical reaction are also included in dry state in the zones of the test strip.

Preferably, the substrates of the enzyme reaction are placed in the reagent pad because they typically have a low molecular weight and are mobile in solution. The enzymes are positioned on the reaction pad because they have a high molecular weight and are relatively immobile. In a very preferred embodiment the reaction pad is composed of a nitrocellulose membrane to which the enzymes will bind so tightly as to be effectively immobilised.

Preferably, the test strip comprises a start zone containing a dried detergent extractant, a reagent zone containing one or more dried enzyme substrates and/or co-factors and a reaction zone containing at least one kind of immobilised enzyme.

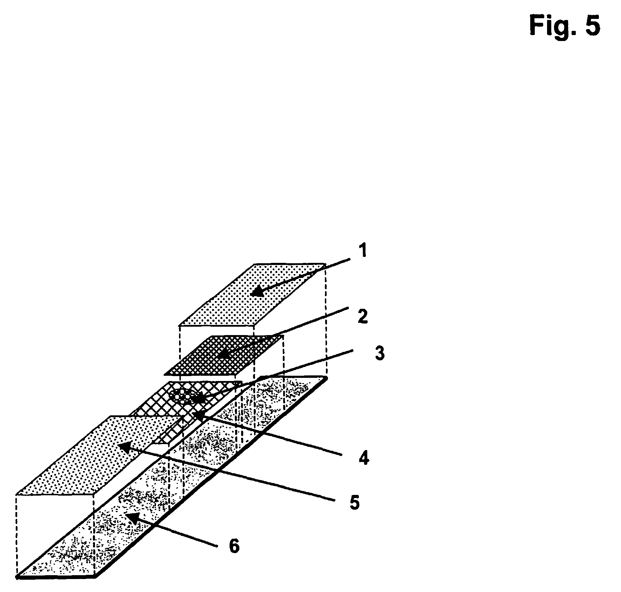

Figure 5 is a schematic, illustrative view of a lateral flow test strip for use according to the present invention. On a self-adhesive plastic base (6) are located a sample pad as start zone (1), a reagent zone (2) partially overlapping with the start zone, a reaction zone (4) with enzyme (3) immobilised on to it. Adjacent to the reaction zone is a wick (5).

This lateral flow test device at least comprises the reagents necessary for the enzymatic conversion of NAD(P) to NAD(P)H, preferably also at least the reagents necessary to detect NAD(P)H by generating a signal.

Preferably, the start zone, composed of an absorbent paper, holds a dried detergent extractant, the reagent zone, also composed of absorbent paper or of glass fibre, contains the substrates for the enzymes used in the assay, while the enzymes themselves are immobilised on the reaction zone, composed of a nitrocellulose membrane with a high protein binding capacity. Preferred reagents for the assay are DTAB (Dodecyl Trimethyl Ammonium Bromide) as detergent extractant on the start zone, the enzymes glucose dehydrogenase and diaphorase immobilised at the reaction zone and the corresponding substrates NBT and glucose dried onto the reagent zone.

Alternatively, DTAB can be incorporated into the substrate reagent dried on to the reagent zone.

Liquid sample applied to the start zone moves by capillary action into the reagent zone, where the enzyme substrates are rehydrated and move with the sample to the enzymes immobilised at the reaction zone. In the presence of the analytes NAD, NADH, NADP and/or NADPH the yellow NBT is converted to the dark blue formazan salt by the action of the enzymes and their substrates, producing a colour change visible by eye. The reduced solubility of the formazan product acts to concentrate it near the reaction site, giving a more intense colour. High sensitivity is achieved because the two enzymes recycle NAD(P) and NAD(P)H, producing many coloured molecules for each analyte molecule present in the sample, providing substrates are supplied from the reagent zone.

Other tetrazolium compounds or diaphorase substrates may be substituted for NBT to produce a readily detectable product with NAD(P)H and diaphorase. In another embodiment the enzyme diaphorase may be replaced by appropriate chemical reagents, for example Meldola's Blue and NBT, which can produce a readily detectable product in the presence of NAD(P)H.

The capacity of the sample pad is such that a single drop of a suitable wetting solution wets the sample pad but not the other components of the test device such that the pad can be used to sample a trial surface without initiating the signal producing reactions. Subsequently a second drop of wetting solution initiates capillary flow thus rehydrating the assay reagents by wetting the entire device, leading to signal production in the presence of NAD(P) or NAD(P)H.

To enable the measurement of two important parameters e.g. to monitor sample hygiene by a single device in the same sample, reagents that produce a colorimetric reaction with protein may be immobilised on the reaction strip at a separate location to the enzymes and substrates used to detect NAD(P) and NAD(P)H. Assay of protein and NAD in the same sample provides additional sensitivity and information regarding the composition of the sample.

As the reagent for detecting protein, those necessary for detection according to the various protein detecting methods, such as biuret reaction method, Lowry method, Coomassie dying method, BCA method and ninhydrin reaction method, are usable in the method of the invention. The reagent for detecting protein is preferably selected from the group of non-octahalogenated sulfophthaleines, desirably from the group consisting of phenol sulfonephthaleins and cresol sulfonephthaleins. Suitable reagents are for instance bromophenol blue, bromocresol green, bromocresol purple, bromophenol red, bromothymol blue, and bromochlorophenol.

Without further elaboration, it is believed that one skilled in the art can, using the preceding description, utilise the present invention to its fullest extent. The preferred specific embodiments and examples are, therefore, to be construed as merely illustrative, and not limiting to the remainder of the disclosure in any way whatsoever.

### Examples

The following examples represent practical applications of the invention.

### Example 1:

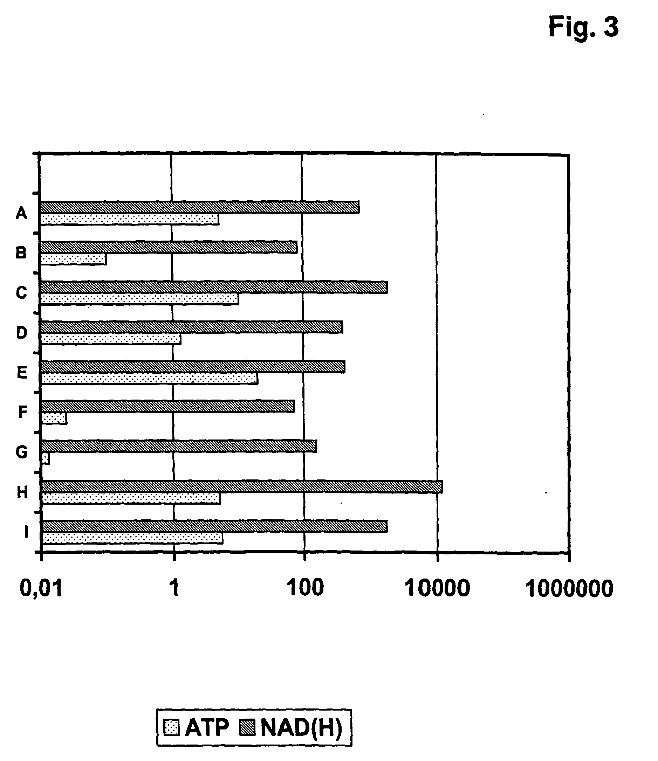

The use of the current invention for the practical detection of food residues is illustrated in figure 3. A variety of common foods homogenised in 1 ml of purified water are used as samples in a colorimetric assay similar to that described in figure 1. Reduction of the tetrazolium salt nitro blue tetrazolium (NBT) is mediated by the enzyme diaphorase. The dehydrogenase used to convert NAD to NADH is glucose dehydrogenase. In this example glucose, diaphorase and a sample consisting of NAD of known concentration (1 µM) or food sample are added to an appropriate buffer (75 mM Tris, 25 mM potassium chloride, 5 mM magnesium acetate, 2 mM EDTA, 0.5 mg/ml BSA, 0.1% Triton X-100, 1.25% (w/v) alpha-cyclodextrin, pH 7.75). The colorimetric reaction is initiated by addition of glucose dehydrogenase.

Formation of coloured formazan product after 5 minutes is measured spectrophotometrically at 540 nm. Comparison with the colour formed under identical conditions by an NAD standard of known concentration allows calculation of the NAD(H) concentration in the original sample. To validate the invention as a practical application it is necessary to show that it is comparable to existing cleanliness assays, for example the HY-LiTE® ATP luminescence assay manufactured by Merck KGaA. To get a fair comparison DTAB was left out in the HY-LiTE® assay. ATP concentrations are measured using this product by first pushing home the sample rod of a standard HY-LiTE® pen to rehydrate the freeze dried firefly reagent and measuring background luminescence using a HY-LiTE® luminometer. The plastic cap of the pen is removed and 30 µl of sample added to the buffer in the main body of the pen and the plastic cap re-attached. The pen is then replaced in the luminometer and the resulting sample luminescence measured. Luminescence measurements are converted to ATP concentrations by calibration using an ATP standard of known concentration in a separate measurement. Comparison of NAD(H) and ATP concentrations found in food samples illustrated in figure 3 indicate that in most cases the NAD(H) content of food is at least equal to that of ATP. Therefore detection of food residues by an NAD(H) assay is demonstrated as being as valid as detection using an ATP assay.
In figure 3, the capital letters stand for the following common foods: A = Boiled Egg, B = Mustard, C = Carrot, D = Cheese, E = Pineapple, F = Port, G = Red Wine, H = Yoghurt, I = Meat. The x-axis shows the nM concentration of ATP or NAD(H).

### Example 2:

The use of the current invention to assess the cleanliness of surfaces by measuring surface nicotinamide adenine dinucleotides is demonstrated in Table 1. A test strip consisting of a plastic support onto which an appropriate paper pad is attached at one end is first wetted using an appropriate solution (6% isopropanol in distilled water). The wet pad is then used to wipe over the test surface (typically a 30 cm² area) transferring sample from the surface to the test strip. The nicotinamide adenine dinucleotides present in the sample are measured by addition of reagents to the test strip producing a coloured product visible to the eye. First one drop (approximately 40 µl) of an appropriate substrate formulation (10 mM sodium citrate pH 6, 2 mg/ml NBT, 300 mM lithium lactate, 0.01% proclin, 2% (w/v) alpha-cyclodextrin) is added to the test strip followed by one drop of an appropriate enzyme formulation (200 mM Tris pH 8, 6 mg/ml diaphorase, 3 mg/ml lactate dehydrogenase, 50% glycerol, 0.01% proclin, 0.3% Tween 80) to initiate the reaction. The samples used in this test are obtained from a domestic kitchen.

The presence of nicotinamide adenine dinucleotides is indicated by the development of a colour on the test strip that can be readily distinguished by eye. The more nicotinamide adenine dinucleotides present the faster colour develops. Colour development is assessed 5 minutes after initiation of the reaction in this example. This time may vary between 1 and 10 minutes depending on the application.

Table 1 illustrates the results obtained from this test. Colour development, indicating a "dirty" surface is indicated by +. No colour development, indicating a "clean" surface, is indicated by -. Rapid colour formation, indicating a "very dirty" surface, is indicated by ++. The results in table 1 indicate that the invention in the form used in this test is capable of detecting residues on an apparently clean surface by a visual colour change. Furthermore the number of unclean surfaces detected is significantly reduced after cleaning indicating that the sensitivity of the invention in this form is correct for this application.

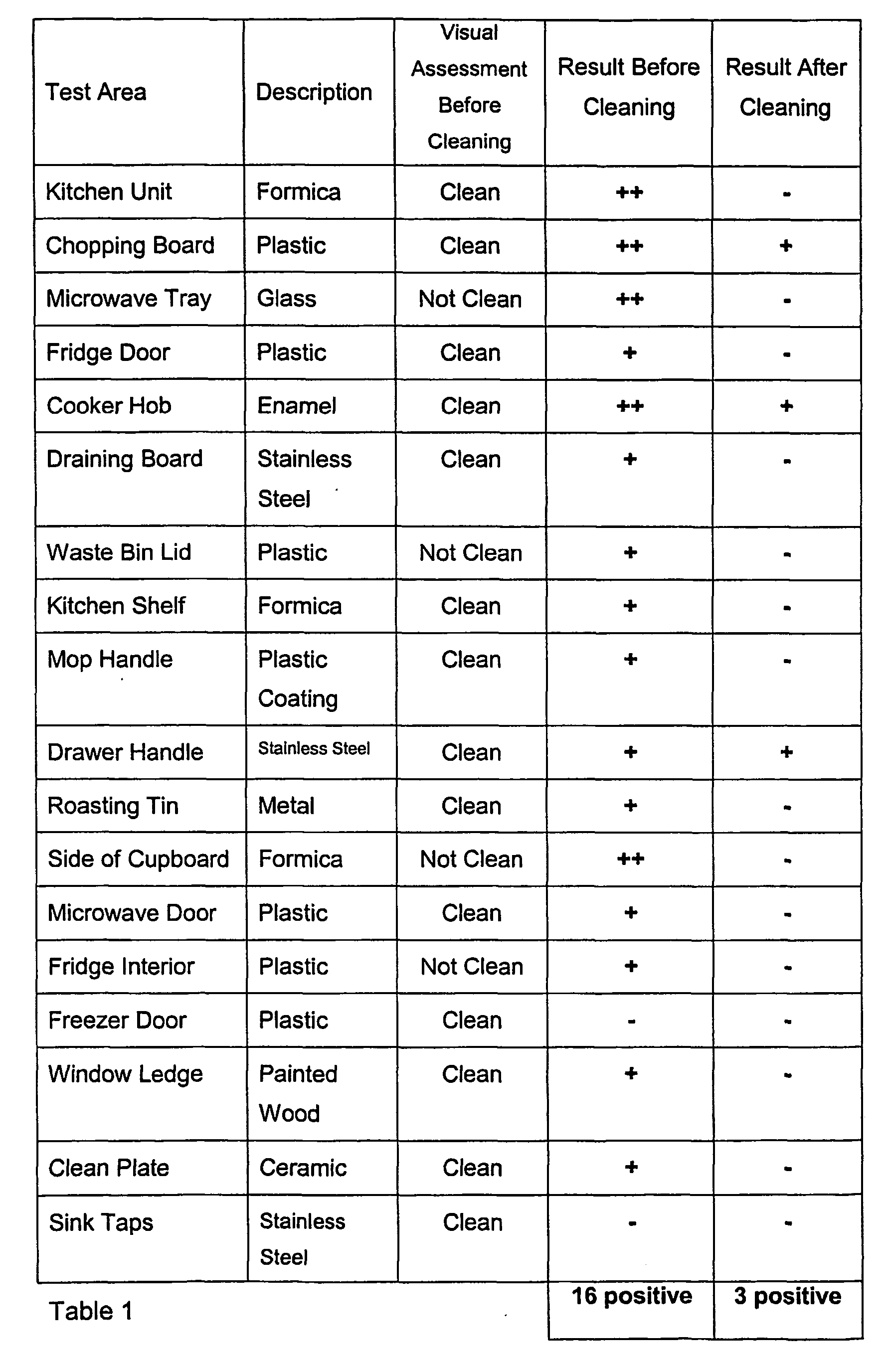

### Example 3:

The use of the current invention to investigate the cleanliness of surfaces in comparison with microbiological hygiene monitoring is illustrated in Table 2. Nicotinamide adenine dinucleotide content as a measure of cleanliness of the surface is measured by the current invention as described in Example 2. Bacterial content on the surface is determined by swabbing a 10 cm x 10 cm area and rinsing the swab into 1 ml of physiological saline. A 100 µl aliquot of the solution is plated onto a Nutrient Agar plate and the number of colonies present after 3 days incubation at 30°C used as a measure of the number of microbes on the surface (CFU / swab). Samples in this example were taken in an institutional food preparation environment.

Table 2 illustrates the relative merits of the methods. The plate count method measures surface contamination of aerobic micro-organisms with the possibility of extending the methodology to identify the types of organism present. Measurement of surface nicotinamide adenine dinucleotide provide information regarding cleanliness/hygiene levels. In other words nicotinamide adenine dinucleotide analysis indicates the level of organic "soil" (biological waste matter) present rather than viable microbial numbers. The presence of soil indicates that a surface has not been thoroughly cleaned and has the potential to provide the conditions for microbial contamination.

**Table 2**

| **Test Area** | **Description** | **Visual Assessment** | **NAD Test** | **Plate Counts** |
|---|---|---|---|---|
| | | | | CFU/swab |
| Carving Knife | Metal | Clean | - | 0 |
| Bread Fridge | Steel | Clean | - | 530 |
| Wash-Up Sink | Steel | Clean | - | 5120 |
| Meat chopping board | Plastic | Clean | - | 10000 |
| Cutlery Tray | Plastic | Clean | + | 0 |
| Milk Dispensing Machine | Plastic | Clean | + | 10 |
| Multi Toaster | Steel | Not Clean | + | 2910 |
| Fryer Lid | Steel | Clean | + | 4220 |
| Cooker hob | Metal | In use | ++ | 0 |
| Microwave Tray | Glass | Not Clean | ++ | 0 |
| Work Surface | Steel | Clean | ++ | 550 |
| Flour container | Plastic | In Use | ++ | 1040 |
| All purpose fridge | Plastic | Not Clean | ++ | 2080 |
| Fresh produce storage shelf | Wood | In use | ++ | 100000 |
| Food preparation unit | Steel | Clean | ++ | 100000 |

The data highlights the advantages of measuring nicotinamide adenine dinucleotides to assess cleanliness. In three cases the plate count method suggests no viable micro-organisms but the presence of nicotinamide adenine dinucleotides, indicating an unclean surface. This demonstrates the ability of this invention to detect biological waste matter. It should also be noted that in all the samples measured in this example the microbial contamination determined by plate counting is below the detection limit of micro-organisms for the form of the invention used in example 4. This indicates that the majority of nicotinamide adenine dinucleotide detected as a marker for poor surface cleanliness is extracellular to bacterial cells. The data also shows that the current invention has the ability to detect levels of nicotinamide adenine dinucleotides on surfaces which are judged to be clean by visual assessment.

Microbiological methods such as plate counts take several days to complete and therefore can only provide a retrospective picture of the surface contamination. Luminometric assessment of ATP requires the use of an instrument for light measurements. This invention takes only minutes, requires no instrumentation and is less subjective than visual assessments. This application of the invention provides the ability to assess surface cleanliness in real time enabling immediate corrective action if required.

### Example 4:

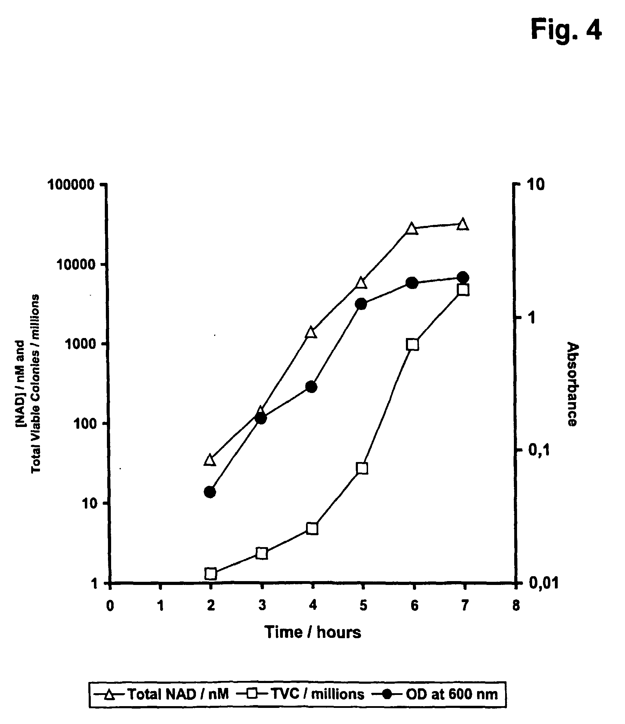

The use of the current invention to determine the growth curve of a bacterial culture and to detect bacteria is illustrated in figure 4. Growth of *E. coli* NCTC 9001 in a culture is initiated by the addition of a sample of a bacterial colony to an appropriate growth medium such as nutrient broth. Bacterial growth in the culture is estimated by taking samples at appropriate times and measuring the optical density at 600 nm, the nicotinamide adenine dinucleotide content using the current invention and the number of total viable colonies (TVC), using a microbial plate count. In this example of the invention a drop of appropriate cell-lysing agent, for example DTAB, followed by a drop of an appropriate substrate mix containing an appropriate neutraliser, for example alpha-cyclodextrin, followed by a drop of an appropriate enzyme solution to initiate the reaction is added to the sample and the rate of colour formation measured using a spectrophotometer. NAD is converted to NADH by the enzyme lactate dehydrogenase with its substrate lithium lactate. Colour is generated from NADH by the enzyme diaphorase and NBT. The rate of colour formation of a sample of known NAD concentration is used to relate rates of colour formation of samples to absolute nicotinamide adenine dinucleotide concentrations.
Figure 4 shows that the current invention can monitor the growth dynamics of a bacterial population to correlate with optical density and total viable colony measurements, the current accepted routine methods of assaying bacterial content.

### Example 5:

The use of the current invention to detect bacteria using a colour change visible by eye can be demonstrated by dipping an appropriate paper pad attached to the end of a plastic strip into a *E. coli* NCTC 9001 culture with a TVC of 5 × 10⁸ cfu/ml, determined by plate count, so that the pad becomes wetted by the culture. To the pad are added sequentially a drop of suitable cell lysing reagent, for example DTAB, a drop of appropriate substrate, for example lithium lactate and NBT, including a neutralising compound, for example alpha-cyclodextrin, and finally a drop of appropriate enzyme formulation, for example lactate dehydrogenase and diaphorase. After 5 minutes significant colour is formed on the pad indicating that this invention can detect bacteria by a visible colour change.

### Example 6:

The use of the current invention with a filter to concentrate bacteria can enhance the sensitivity of micro-organism detection. As an example 20 ml of a culture containing 6 × 10 ⁵ cfu / ml of *E. coli* NCTC 9001 is passed through a nylon filter of 0.2 µm pore size. To this filter is added sequentially one drop (approximately 40 µl) of a cell-lysing agent (for example DTAB), one drop of substrate solution containing an appropriate neutraliser (for example lithium lactate, NBT and alpha-cyclodextrin) and finally one drop of enzyme solution (for example lactate dehydrogenase and diaphorase). Colour clearly visible by eye develops on the filter within 7 minutes. When the experiment is repeated using only a single drop of the *E. coli* culture containing 6 × 10 ⁵ cfu / ml as sample on the filter no colour visible by eye is formed on addition of cell lysing reagent, substrate and enzyme solutions, even after 30 minutes. This example demonstrates that by concentrating the microbial content of a solution on a filter on which a colorimetric assay can be performed an increase in sensitivity can be obtained.

### Example 7:

The use of the current invention with a heating block to enhance the rate of reaction and decrease time required for reading is illustrated by performing an assay in which one drop of NAD of known concentration (i.e. ca. 40µl of a 1,25 µM solution) is added to a test strip, followed by one drop of a substrate solution consisting of lithium lactate and NBT and finally one drop of enzyme solution consisting of lactate dehydrogenase and diaphorase. The time taken for a "positive" result estimated by eye at 20 °C (ambient temperature) is 3.5 min. When the experiment is repeated at 40 °C by placing the strip on a dedicated thermostatically-controlled heating block the time taken for a "positive" result estimated by eye is only 1 min.

In a second example, samples of NAD of a known concentration, substrate solution and enzyme solution as described above are placed in two appropriate cuvettes, incubated at temperatures of 18 °C and 43 °C respectively and the absorbance measured at 540 nm after 5 minutes. Absorbance at 18 °C was 1.072 while that at 43 °C was 2.256, an increase in the rate of colour formation of 210%.
These results indicate that significant improvements in the assay sensitivity are possible by performing the assay at temperatures greater than ambient, attained using either a purpose-built heating block or any appropriate incubator.

### Example 8 - Lateral Flow test device: NAD

A lateral flow test device, comprising a nitrocellulose reaction strip, paper sample pad, reagent pad and wick is constructed as shown in figure 5. The reagent pad is soaked in a solution of substrate formulation comprising 450 mM glucose, 2 mg/ml NBT, 10 mM citrate and then dried prior to assembly. Devices are based on the Millipore SR membrane, which comprises a nitrocellulose membrane on an inert plastic support. Self-adhesive portions on the plastic support allow attachment of suitable paper strips to act as sample pad, reagent pad and wick. Devices are constructed as 20 cm wide strips which are cut into individual devices with a width of 5 mm.

To prepare the strips for use 1 µl of enzyme formulation comprising 200 mM Tris, 3 mg/ml diaphorase, 8 mg/ml glucose dehydrogenase is added to the nitrocellulose portion of the strip and allowed to dry at 20 °C for 10 minutes. In this configuration enzyme and substrate formulations are kept separate, improving the long term stability of the device. In addition each 5 mm width of reagent pad contains the equivalent of approximately 20 µl of substrate, ensuring that the immobilised enzyme on the reaction strip has sufficient substrate for the complete time course of the reaction.

The assay is activated by the addition of a solution containing 15 pmoles of NAD to the sample pad. The sample moves into the reagent pad where the enzyme substrates are rehydrated and carried with the sample into the nitrocellulose reaction pad. Here NAD, assay enzymes and substrates react to produce purple formazan product visible by eye within 5 minutes at 20 °C, indicating that this device is capable of detecting NAD in solution. When the experiment is repeated using a distilled water control instead of an NAD solution as sample, no purple colour is formed after 10 minutes at 20 °C.

### Example 9 - Lateral Flow test device: Biomass

To demonstrate the use of the lateral flow test device to detect biomass, 30 µl of a broth culture of *E.coli* NovaBlue (Novagen, Catalogue No.69009 -3) containing 7.2 × 10⁷ colony forming units / ml (determined by total aerobic microbiological plate count) is added to the sample pad of a strip prepared as described in example 8. To activate the device 30 µl of an extractant solution consisting of 0.3 % w/v DTAB in water is added to the sample pad, providing sufficient volume for the liquid sample to move through the device by capillary action. The extractant lyses the bacteria present on the sample pad, allowing intracellular NAD(P) and NAD(P)H to move to the reaction pad via the reagent pad with the mobile sample phase. Purple colour indicating the presence of NAD(P)(H), equivalent to a "dirty" result in a hygiene monitoring assay, is obtained within 5 minutes. When the experiment is repeated using sterile broth containing no bacteria, no colour formation is observed.

## Claims

1. A method for detecting the presence of biomass in a sample based on the detection of the total pool of NAD(P) and NAD(P)H, **characterised in that** the total pool of NAD(P) and NAD(P)H is measured as a marker for the presence of biomass by conversion of NAD(P) in the sample to NAD(P)H enzymatically by adding an enzyme E1 and a substrate S1 for that enzyme.

2. A method according to claim 1, **characterised in that** the biomass present is detected and measured.

3. A method according to claims 1 or 2, **characterised in that** the detection is done fluorimetrically.

4. A method according to claims 1 or 2, **characterised in that** the detection is done by conversion of NAD(P) H to NAD(P) at an electrode surface leading to a change in potential or current at an electrode surface.

5. A method according to claims 1 or 2, **characterised in that** the detection is done by adding a second enzyme E2 and a second substrate S2, whereby the following reaction takes place 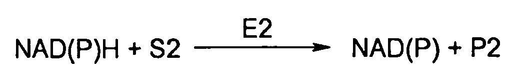 and the conversion of S2 into P2 generates a signal which is a change in the absorbance spectrum including a change in colour, a change in fluorescence, a luminescent reaction or an electrochemical reaction leading to a change in potential at an electrode surface.

6. A method according to claim 5, **characterised in that** the substrates S1 and S2 are added to the sample in excess.

7. A method according to claim 5 or 6 **characterised in that** E1 is lactate dehydrogenase, E2 is diaphorase, S1 is lithium lactate and S2 is nitro blue tetrazolium.

8. A method according to claim 5 or 6 **characterised in that** E1 is glucose dehydrogenase, E2 is diaphorase, S1 is glucose, S2 is nitro blue tetrazolium.

9. A method according to one or several of claims 1 to 8, **characterised in that** the sample is heated at a temperature between 25 and 45 °C.

10. A method according to one or several of claims 1 to 9, **characterised in that** prior to detecting the amount of biomass a detergent and optionally afterwards a neutraliser are applied to the sample.

11. Use of the method according to one or several of claims 1 to 10 for the quantitation and/or detection of microorganisms or the hygiene status of a sample.

## Patentansprüche

1. Verfahren zum Nachweisen des Vorliegens von Biomasse in einer Probe auf Basis des Nachweises des gesamten Pools von NAD(P) und NAD(P)H, **dadurch gekennzeichnet, dass** der gesamte Pool von NAD(P) und NAD(P)H als Marker für das Vorliegen von Biomasse gemessen wird, indem NAD(P) in der Probe durch Zugabe eines Enzyms E1 und eines Substrats S1 für das Enzym enzymatisch in NAD(P)H umgewandelt wird.

2. Verfahren nach Anspruch 1, **dadurch gekennzeichnet, dass** die vorliegende Biomasse nachgewiesen und gemessen wird.

3. Verfahren nach den Ansprüchen 1 oder 2, **dadurch gekennzeichnet, dass** der Nachweis fluorimetrisch erfolgt.

4. Verfahren nach den Ansprüchen 1 oder 2, **dadurch gekennzeichnet, dass** der Nachweis anhand der Umwandlung von NAD(P)H in NAD(P) an einer Elektrodenoberfläche erfolgt, was zu einer Änderung im Potenzial oder im Strom an einer Elektrodenoberfläche führt.

5. Verfahren nach den Ansprüchen 1 oder 2, **dadurch gekennzeichnet, dass** der Nachweis durch Zugabe eines zweiten Enzyms E2 und eines zweiten Substrats S2 erfolgt, wodurch die folgende Umsetzung stattfindet: 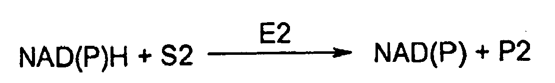 und die Umwandlung von S2 in P2 ein Signal erzeugt, das eine Veränderung im Absorptionsspektrum ist, einschließlich einer Farbänderung, einer Fluoreszenzänderung, einer Lumineszenzreaktion oder einer elektrochemischen Reaktion, die zu einer Änderung im Potenzial oder im Strom an einer Elektrodenoberfläche führt.

6. Verfahren nach Anspruch 5, **dadurch gekennzeichnet, dass** die Substrate S1 und S2 im Überschuss zur Probe gegeben werden.

7. Verfahren nach Anspruch 5 oder 6, **dadurch gekennzeichnet, dass** E1 Lactatdehydrogenase ist, E2 Diaphorase ist, S1 Lithiumlactat ist und S2 Nitro-Blau-Tetrazolium ist.

8. Verfahren nach Anspruch 5 oder 6, **dadurch gekennzeichnet, dass** E1 Glucosedehydrogenase ist, E2 Diaphorase ist, S1 Glucose ist und S2 Nitro-Blau-Tetrazolium ist.

9. Verfahren nach einem oder mehreren der Ansprüche 1 bis 8, **dadurch gekennzeichnet, dass** die Probe auf eine Temperatur zwischen 25 und 45°C erwärmt wird.

10. Verfahren nach einem oder mehreren der Ansprüche 1 bis 9, **dadurch gekennzeichnet, dass** vor dem Nachweisen der Menge an Biomasse ein Detergenz und gegebenenfalls anschließend ein Neutralisationsmittel zur Probe gegeben werden.

11. Verwendung des Verfahrens nach einem oder mehreren der Ansprüche 1 bis 10 zur Quantifizierung und/oder zum Nachweis von Mikroorganismen oder des Hygienestatus einer Probe.

## Revendications

1. Procédé pour détecter la présence d'une biomasse dans un échantillon sur la base de la détection de l'ensemble total de NAD(P) et de NAD(P)H, **caractérisé en ce que** l'ensemble total de NAD(P) et de NAD(P)H est mesuré en tant que marqueur quant à la présence d'une biomasse par conversion de NAD(P) dans l'échantillon selon NAD(P)H de manière enzymatique en ajoutant un enzyme E1 et un substrat S1 pour cet enzyme.

2. Procédé selon la revendication 1, **caractérisé en ce que** la biomasse présente est détectée et est mesurée.

3. Procédé selon la revendication 1 ou 2, **caractérisé en ce que** la détection est réalisée fluorimétriquement.

4. Procédé selon la revendication 1 ou 2, **caractérisé en ce que** la détection est réalisée par conversion de NAD(P)H selon NAD(P) au niveau d'une surface d'électrode aboutissant à la modification de potentiel ou de courant au niveau d'une surface d'électrode.

5. Procédé selon la revendication 1 ou 2, **caractérisé en ce que** la détection est réalisée en ajoutant un second enzyme E2 et un second substrat S2 et ainsi, la réaction qui suit est réalisée 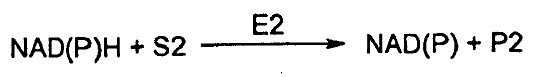 et la conversion de S2 selon P2 génère un signal qui est une modification du spectre d'absorbance incluant une modification de couleur, une modification de fluorescence, une réaction luminescente ou une réaction électrochimique aboutissant à une modification du potentiel au niveau d'une surface d'électrode.

6. Procédé selon la revendication 5, **caractérisé en ce que** les substrats S1 et S2 sont ajoutés à l'échantillon en excès.

7. Procédé selon la revendication 5 ou 6, **caractérisé en ce que** E1 est déshydrogénase de lactate, E2 est diaphorase, S1 est lactate de lithium et S2 est nitro-bleu de tétrazolium.

8. Procédé selon la revendication 5 ou 6, **caractérisé en ce que** E1 est déshydrogénase de glucose, E2 est diaphorase, S1 est glucose et S2 est nitro-bleu de tétrazolium.

9. Procédé selon une ou plusieurs des revendications 1 à 8, **caractérisé en ce que** l'échantillon est chauffé à une température entre 25°C et 45°C.

10. Procédé selon une ou plusieurs des revendications 1 à 9, **caractérisé en ce que**, avant la détection de la quantité de biomasse, un détergent et en option ensuite un neutraliseur sont appliqués sur l'échantillon.

11. Utilisation du procédé selon une ou plusieurs des revendications 1 à 10 pour la quantification et/ou la détection de microorganismes ou de l'état d'hygiène d'un échantillon.
